# EUROPÄISCHE PATENTSCHRIFT

(11) **EP 2 663 630 B1**
(45) Veröffentlichungstag und Bekanntmachung des Hinweises auf die Patenterteilung: **14.10.2020**
(21) Anmeldenummer: 12700645.0
(22) Anmeldetag: 11.01.2012
(51) Int. Cl.: C12M 1/113, C12M 1/107

(54) **RÄUMER FÜR EINEN FERMENTER ZUM FÖRDERN VON RÄUMGUT, FERMENTER UND VERFAHREN ZUM RÄUMEN VON RÄUMGUT**
SCRAPER FOR A FERMENTER FOR CONVEYING SCRAPED MATERIAL, FERMENTER, AND METHOD FOR SCRAPING SCRAPED MATERIAL
RACLEUR POUR UN FERMENTEUR POUR TRANSPORTER DES MATIÈRES À ÉVACUER, FERMENTEUR ET PROCÉDÉ POUR ÉVACUER DES MATIÈRES À ÉVACUER

(30) Priorität: 11.01.2011 DE 102011008319
(43) Veröffentlichungstag der Anmeldung: 20.11.2013
(73) Patentinhaber: Finsterwalder Umwelttechnik GmbH & Co. KG, 83233 Bernau a. Chiemsee/Hittenkirchen (DE)
(72) Erfinder: FINSTERWALDER, Klemens, 82335 Berg (DE); FINSTERWALDER, Tobias, 80469 München (DE)
(74) Vertreter: Molnia, David
(86) Internationale Anmeldenummer: PCT/EP2012/050382
(87) Internationale Veröffentlichungsnummer: WO 2012/095462

(56) Entgegenhaltungen:
- EP-A1- 0 182 955
- DE-A1- 2 544 177
- DE-A1- 2 634 632
- DE-A1- 3 617 385
- DE-C1- 19 621 914
- DE-C1- 19 815 842
- DE-U1- 20 008 186
- DE-U1- 20 011 783
- DE-U1-202009 015 099
- US-A- 2 326 303

## Beschreibung

### Technisches Gebiet

Die Erfindung betrifft einen Räumer für einen Fermenter zum Fördern von Räumgut, insbesondere von Sinkstoffen, zu einem Auswurfbereich, sowie einen Fermenter und ein Verfahren zum Räumen von Räumgut in einem Fermenter.

### Technischer Hintergrund

In einer Biogasanlage, insbesondere einer anaeroben Vergärungsanlage, werden organische Bestandteile in einem geschlossenen Fermenterbehälter, der als Fermenter bezeichnet wird, durch Bakterien zersetzt, also fermentiert. Bei diesem Prozess entsteht das brennbare Gas Methan, welches auch "Biogas" genannt wird. In einem Biogasheizkraftwerk kann das Biogas dann zur Energiegewinnung verbrannt werden.

Biogasanlagen werden üblicherweise mit nassen Bioabfällen beschickt, welche hauptsächlich Abfallbestandteile organischen Ursprungs enthalten, wie beispielsweise Gemüse, Obst, Knochen und/oder Fleischreste. In dem Abfall befinden sich aber typischerweise und unvermeidbar auch Störstoffe, wie beispielsweise Metalle, Verpackungsstoffe (z.B. Folien, Netze, Schaumstoffe und Verschlüsse) sowie Mineralstoffe (beispielsweise Tellerscherben, Sand oder Kies), die dem eigentlichen Fermentierungsprozess nicht zugänglich sind.

Die Abfälle befinden sich in einem Fermenter typischerweise in wässriger Suspension, wobei sich die Bioabfallsuspension in drei Fraktionen aufteilt, nämlich in Sinkstoffe, die auf dem Behälterboden absinken, Schwebstoffe, die in der Suspension im Gleichgewicht gehalten werden, und Schwimmstoffe, die auf der Oberfläche der Bioabfallsuspension aufschwimmen.

Nach der vollständigen Fermentation der organischen Bestandteile können die organisch nicht mehr abbaubaren Reststoffe dann als Flüssig- oder Festkompost verwertet werden.

Die DE 198 15 842 C1 beschreibt eine Biogasanlage zur Behandlung, Aufbereitung und Verwertung unsortierter, überwiegend nasser und organischer Abfälle. Gemäß dieser Offenbarung wird das in den Fermenter eingebrachte Abfallgemisch in drei Fraktionen unterschiedlicher Dichte, nämlich in Sinkstoffe, Schwebstoffe und Schwimmstoffe, aufgeteilt, wobei deren jeweilige Verweilzeiten im Fermenter steuerbar sind, da die unterschiedlichen Fraktionen jeweils individuell aus dem Fermenterbehälter ausgetragen werden können.

Die leichteste Fraktion, nämlich die auf der wässrigen Suspension aufschwimmenden Schwimmstoffe, können durch Abschöpfen aus dem Fermenterbehälter ausgetragen werden.

Die zweitdichteste Fraktion, nämlich die wässrige Suspension der Schwebstoffe, wird im Fermenterbehälter in Rotation gebracht und in Bewegung gehalten, wobei die Bewegungsgeschwindigkeit durch Steuerung des entsprechenden Rührers steuerbar ist. Über die Rotationsgeschwindigkeit kann eingestellt werden, bis zu welcher Partikelgröße bzw. Partikelmasse Partikel in Suspension gehalten werden können.

Die dichteste Fraktion, nämlich die Sinkstoffe, sinken auf den Boden des Fermenters ab und werden mittels eines mit einer Vielzahl von Lamellen versehenen Räumers zu einer Austragsschnecke transportiert und von dieser aus dem Fermenterbehälter entfernt.

Das in der DE 198 15 842 C1 beschriebene System zum Räumen der Sinkstoffe funktioniert solange gut, wie das Räumgut, also die Sinkstoffe, einen hohen Dichteunterschied zur Umgebung aufweisen und solange keine Zopfbildung im Fermenterbehälter auftritt. Zopfbildung tritt in dem Fermenterbehälter durch Verknoten oder Verknäulen faserförmiger Reststoffe oder von Plastikteilen beispielsweise an den Lamellen des Räumers auf.

DE 200 08 186 U offenbart eine Räum-und Austrageinrichtung für Sinkschichten an einem Fermenter zur anaeroben Fermentation von organischen Stoffen. DE 20 2009 015099 U offenbart einen Räumer für einen Fermenter, der verbackene Biomasse-Klumpen in der äußeren Peripherie erfasst, aufbricht und in den konischen unteren Teil des Vorratsbehälters fördert.

### Zusammenfassung der Offenbarung

Aufgabe der vorliegenden Erfindung ist es, einen Räumer bereitzustellen, der verbesserte Eigenschaften bezüglich des Austrags des Räumgutes aufweist und die Zopfbildung reduziert. Diese Aufgabe wird durch einen Räumer für einen Fermenter zum Fördern von Räumgut mit den Merkmalen des Anspruchs 1 gelöst. Entsprechend weist der Räumer eine Räumvorrichtung auf, welche sich von einer im Zentrum eines Fermenters anordenbaren Drehachse aus erstreckt. Erfindungsgemäß weist die Räumvorrichtung eine in einer senkrecht zu der Drehachse liegenden Ebene gekrümmte Räumkante auf.

Unter der Räumkante wird diejenige Kante der Räumvorrichtung verstanden, welche mit dem Räumgut zuerst in Kontakt tritt welche dieses vor sich her schiebt. Die Ausbildung dieser Räumkante in einer gekrümmten Form ermöglicht es, durch eine einfache Rotation der Räumvorrichtung um die im Zentrum des Fermenters angeordnete Drehachse herum das Räumgut kontinuierlich entlang der Räumkante zu transportieren. Auf diese Weise lässt sich ein effizienter Austrag des Räumguts aus dem Fermenterbehälter erreichen.

Weiterhin wird durch die kontinuierliche Räumkante der Räumvorrichtung erreicht, dass diese gegenüber Faserstoffen und Zöpfen unempfindlich ist. Die gekrümmte Räumkante ersetzt die im Stand der Technik üblichen Räumlamellen, die wesentlich kleinteiliger ausgebildet sein müssen und daher einen idealen Anhaftungsort für Fasermaterialien ausbilden, wodurch die unerwünschte Zopfbildung gefördert wird.

Bevorzugt ist die Drehachse in Form einer Antriebswelle ausgebildet, von welcher die Räumvorrichtung angetrieben wird und welche einen Umfang derart aufweist, dass eine Zopfbildung vermieden wird. Entsprechend muss der Umfang der Antriebswelle länger als die längsten in der Suspension vorliegenden Fasern sein, um die Zopfbildung zu vermeiden. Bevorzugt bewegt sich der Durchmesser der Antriebswelle in einem Bereich von 150 mm bis 350 mm.

Die Räumvorrichtung ist in Form eines gekrümmten Räumarmes ausgebildet, wodurch eine technische einfache, schlanke und effiziente Ausbildung der gekrümmten Räumkante erreicht werden kann.

Um eine konstante Förderung des Räumgutes entlang der Räumkante zu erreichen, ist die Räumkante derart gekrümmt, dass der Winkel zwischen einem von der Drehachse ausgehenden Radiusstrahl und der an dem entsprechenden Schnittpunkt des Radiusstrahls mit der Räumkante anliegenden Tangente über die Räumkante hinweg im Wesentlichen konstant ist.

Bevorzugt handelt es sich bei der Form der Krümmung um eine logarithmische Spirale. Bei einer logarithmischen Spirale schneiden alle durch den Pol gehenden Graden die Kurve unter dem gleichen Tangentenwinkel.

Bevorzugt ist der Winkel zwischen dem von der Drehachse ausgehenden Radiusstrahl und der an dem entsprechenden Schnittpunkt des Radiusstrahls mit der Räumkante anliegenden Tangente in einem Bereich zwischen 20 und 60 Grad, bevorzugter zwischen 30 und 40 Grad, noch bevorzugter 35 Grad, gewählt, so dass eine kontinuierliche Förderung des Räumgutes stattfindet. Insbesondere muss der Winkel so gewählt werden, dass durch den entstehenden tangentialen Materialvorschub die Reibung zwischen dem Räummaterial und der Räumkante der Räumvorrichtung überwunden wird, damit eine zuverlässige Förderung des Räumgutes stattfinden kann, ohne dass sich Räumgut an einer Stelle der Räumvorrichtung besonders ablagert oder verfängt.

Um das Austragen des Räumgutes zu vereinfachen, beispielsweise mittels mindestens eines an einem Ort im Boden eines Fermenterbehälters vorgesehenen Auswurfbereichs, ist erfindungsgemäß an dem der Drehachse abgewendeten Ende der Räumvorrichtung vorteilhaft mindestens eine Fangvorrichtung vorgesehen, mittels welcher durch die Krümmung der Räumkante nach außen transportiertes Räumgut aufgefangen und bei der Umdrehung mitgeführt werden kann. Die Fangvorrichtung ist so positioniert dass sie mit mindestens einem im Fermenterboden vorgesehenen Auswurfbereich korreliert, wenn dieser von der Räumvorrichtung überstrichen wird. Entsprechend wird das in der Fangvorrichtung geförderte Räumgut beim Überstreichen des Auswurfbereiches zuverlässig ausgetragen. Durch die Fangvorrichtung kann weiterhin erreicht werden, dass nur in einem räumlich begrenzten Bereich ein Auswurfbereich vorgesehen werden muss, da das Räumgut einer Umdrehung der Räumvorrichtung zuverlässig in der Fangvorrichtung transportiert wird, bis es über den Auswurfbereich ausgetragen werden kann.

Die Räumkante der Räumvorrichtung ist bevorzugt so ausgelegt, dass das Räumgut, auf welches die Räumkante auftrifft, stets entlang der Räumkante gefördert wird wenn die Räumvorrichtung um die Drehachse herum bewegt wird.

Der Räumer umfasst vorteilhaft weiterhin eine Absaugvorrichtung, welche mit einem im Boden eines Fermenterbehälters angeordneten mindestens einen Auswurfbereich verbindbar ist. Die Absaugvorrichtung ist bevorzugt so mit der Räumkante korreliert, dass das durch die Räumkante geförderte Räumgut in die Absaugvorrichtung eingetragen wird und von dieser abgesaugt wird. Die Absaugvorrichtung kann entsprechend kontinuierlich betrieben werden, oder sie kann intermittierend mit dem Überstreifen der Räumkante synchronisiert betrieben werden.

Der Räumer umfasst weiterhin bevorzugt einen Schwerkraftabscheider, der mit der Absaugvorrichtung kommuniziert, wobei der Schwerkraftabscheider ein Abscheidevolumen und einen ersten Ablauf zum Ablaufen eines Teilstromes, der von schweren Sinkstoffen befreit ist, umfasst, und wobei ein zweiter Ablauf zum Ablaufen eines von leichteren Sinkstoffen befreiten Teilstroms vorhanden ist, wobei das Aufteilungsverhältnis der beiden Teilströme mittels eines Wehrs eingestellt werden kann. Auf diese Weise kann in dem Schwerkraftabscheider das Räumgut, insbesondere die Sinkstoffe, abgezogen werden. Das Überschusswasser und die feineren Schwebstoffe jedoch, die beim Absaugen des Räumguts zwangsläufig mitgefördert werden, können dem Fermenterbehälter wieder zugeführt werden. Auf diese Weise können die Schwebstoffe weiter fermentiert werden, wodurch eine effiziente Ausnutzung des organischen Materials zur Erzeugung von Biogas in dem Fermenterbehälter erreicht werden kann, aber gleichzeitig ein effizientes Austragen der Sinkstoffe erreicht wird.

Der erste Ablauf ist bevorzugt unterhalb des zweiten Ablaufes in dem Schwerkraftabscheider positioniert, um die von Sinkstoffen weitgehend befreiten Teilströme über ein Gefälle wieder zusammenzuführen und gemeinsam wieder dem Fermenter zuzuführen.

Ein Fermenter mit einem Fermenterbehälter löst ebenfalls die oben angegebene Aufgabe, wenn ein Räumer gemäß der vorstehenden Beschreibung am Boden des Fermenterbehälters vorgesehen ist. Die Drehachse ist im Zentrum des Fermenterbehälters angeordnet und die Räumvorrichtung erstreckt sich von der Drehachse aus in Richtung des Randes des Fermenterbehälters. Die Räumvorrichtung ist um die Drehachse herum rotierbar.

Weiterhin ist ein Verfahren zum Fördern von Räumgut in einem Fermenter zu einem Auswurfbereich mit einem Räumer gemäß der vorstehenden Beschreibung vorgesehen, wobei das Räumgut durch Rotation der Räumvorrichtung entlang der Räumkante gefördert wird.

### Kurze Beschreibung der Zeichnungen

Im Folgenden wird die vorliegende Offenbarung anhand der Zeichnungen der Figuren beschrieben, wobei:
- Figur 1: eine schematische perspektivische Ansicht eines Räumers gemäß der vorliegenden Offenbarung ist;
- Figur 2: schematisch die Geometrie der Räumkante des Räumers zeigt;
- Figur 3: eine schematische dreidimensionale Ansicht eines Schwerkraftabscheiders gemäß der vorliegenden Offenbarung ist;
- Figur 4: eine schematische Schnittdarstellung durch den Schwerkraftabscheider der Figur 3 darstellt; und
- Figur 5: eine Draufsicht entlang der Linie A-A in Figur 4 ist.

### Ausführliche Beschreibung der Figuren

Im Folgenden wird die vorliegende Offenbarung anhand der Figuren ausführlich beschrieben. Dabei werden die gleichen Bezugszeichen für gleiche oder ähnliche Elemente bzw. Komponenten in den Figuren verwendet und teilweise wird auf eine wiederholte Beschreibung verzichtet, um Redundanzen zu vermeiden.

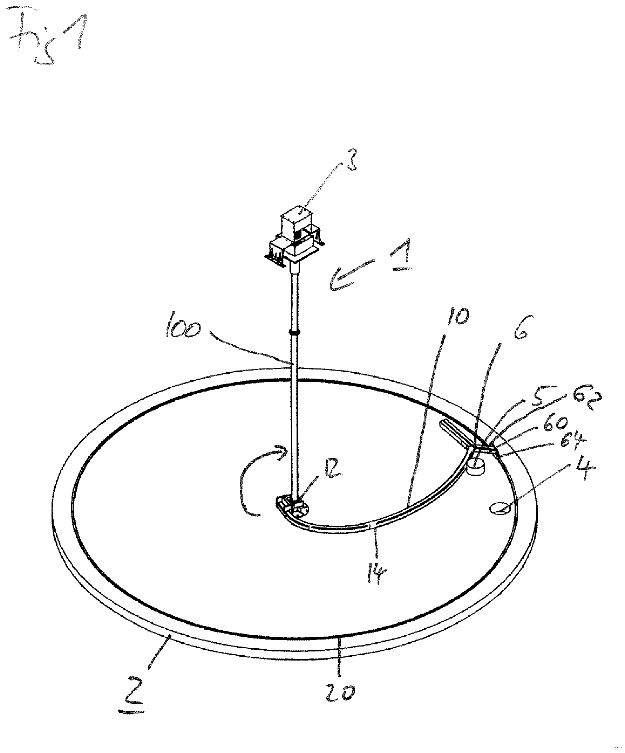

Figur 1 zeigt einen Räumer 1, der auf einem Behälterboden 2 eines Fermenterbehälters angeordnet ist. Der Räumer 1 umfasst eine Räumvorrichtung 10, welche sich von einer Drehachse 100 aus nach außen zum Rand 20 des Bodens 2 des Fermenterbehälters hin erstreckt. Die Drehachse 100 liegt im Zentrum des Fermenterbehälters, wobei die Räumvorrichtung 10 um die Drehachse 100 herum rotieren kann.

Zum Antrieb der Räumvorrichtung 10 ist in dem in Figur 1 gezeigten Ausführungsbeispiel ein Motor 3 vorgesehen, der die Drehachse 100 als Antriebswelle für die Räumvorrichtung 10 verwendet. Die Antriebswelle hat eine Radius von 320 mm, kann aber je nach Faserlänge entsprechend angepasst werden, um die Zopfbildung zu reduzieren oder gar zu verhindern.

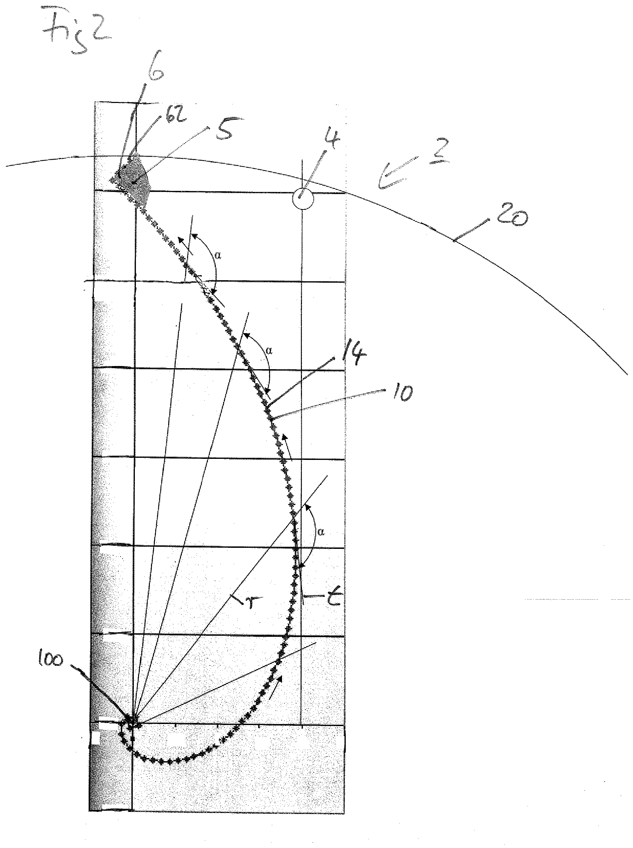

Die Räumvorrichtung 10 weist in der zu der Drehachse 100 senkrecht liegenden Ebene, welche durch den Anflanschbereich 12 der Räumvorrichtung 10 an der Antriebswelle 100 hindurch geht, eine gekrümmte Räumkante 14 auf. Über die genaue Form der Räumkante 14 wird nachfolgend unter Bezugnahme auf Figur 2 näheres erläutert werden.

Im Boden 2 des Fermenterbehälters ist ein Auswurfbereich 4 vorgesehen, durch welchen die sich auf dem Boden 2 des Fermenterbehälters absetzenden Sinkstoffe ausgetragen werden können. In bevorzugten Ausführungsformen sind mehrere Auswurfbereiche über den Umfang verteilt vorgesehen, um das Auswerfen des Räumguts effizient durchführen zu können. Das Räumgut in Form der Sinkstoffe ist in Figur 1 exemplarisch und schematisch durch den Puck 5 gezeigt.

Wenn sich die Räumvorrichtung 10 in Pfeilrichtung um die Drehachse 100 dreht, streicht sie über den Boden 2 des Fermenterbehälters. Hierbei tritt das Räumgut in Kontakt mit der Räumkante 14 und wird entlang der gekrümmten Räumkante 14 in Richtung der Außenseite der Räumvorrichtung 10 transportiert, um dann schließlich über den Auswurfbereich 4 aus dem Fermenterbehälter entfernt zu werden.

An dem der Drehachse 100 abgewandten Ende der Räumvorrichtung 10 ist eine Fangvorrichtung 6 vorgesehen. Die Fangvorrichtung 6 weist eine V-förmige Kante 60 auf, welche dazu vorgesehen ist, das Räumgut 5 aufzunehmen und während der Umdrehungsbewegung der Räumvorrichtung 10 zu speichern. In einer weiteren Ausführungsform können mehrere Fangvorrichtungen vorgesehen sein, um den Räumer zu entlasten. Das Räumgut 5 wird dann mittels der Fangvorrichtung 6 automatisch über der jeweiligen Auslassöffnung 4 zentriert, wenn die Räumvorrichtung 10 und mit dieser die Fangvorrichtung 6 den Auswurfbereich 4 überstreicht.

Die Fangvorrichtung 6 erstreckt sich bis zum äußeren Rand 20 des Fermenterbehälters. In der gezeigten Ausführungsform ist am äußersten, der Drehachse 100 abgelegenen Ende der Räumvorrichtung 10 an dem äußersten Schenkel 62 der Fangvorrichtung 6 ein Räumschild 64 vorgesehen, welches entlang der in Figur 1 nicht gezeigten Fermenterbehälterwand streicht, um an der Fermenterbehälterwand anhaftende Sinkstoffe ebenfalls in Richtung der Fangvorrichtung 6, hier nach innen, zu bewegen.

In Figur 1 ist die Räumvorrichtung 10 in Form eines gekrümmten Räumarmes gezeigt. Der gekrümmte Räumarm 10 erstreckt sich ausgehend von der Drehachse 100 in Richtung der Außenkante 20 des Fermenterbehälters. Er erstreckt sich aber nicht bis ganz an die Wand, sondern nur bis zur Fangvorrichtung 6. Die Fangvorrichtung 6, bzw. deren äußerster Schenkel 62 wird dann bis zur Außenseite 20 des Fermenterbehälters geführt.

An dem Auswurfbereich 4 kann eine Absaugvorrichtung, die in Figur 1 nicht gezeigt ist, angeflanscht sein, um das von der Fangvorrichtung 6 transportierte Räumgut 5 abzusaugen. Die Absaugvorrichtung wird dabei bevorzugt nur dann eingeschaltet, wenn die Räumvorrichtung 10 bzw. die Fangvorrichtung 6 im Begriff ist, die Auslassöffnung 4 zu überstreichen. Entsprechend wird die Absaugvorrichtung im Wesentlichen intermittierend betrieben.

In Figur 2 ist schematisch eine bevorzugte Form eines Räumers 10, bzw. einer gekrümmten Räumkante 14 in Draufsicht gezeigt. Dabei erstreckt sich der Räumer 10, ausgehend von der im Zentrum des Fermenterbehälters 2 angeordneten Drehachse 100 aus, derart, dass er die gekrümmte Räumkante 14 aufweist.

In der in Figur 2 gezeigten bevorzugten Ausführungsform ist der Winkel α zwischen dem jeweiligen, von der Drehachse 100 ausgehenden Radiusstrahl r und der an dem jeweiligen Schnittpunkt zwischen dem Radiusstrahl r und der Räumkante 14 anliegenden Tangente c über die gesamte gekrümmte Räumkante hinweg konstant. Ein Beispiel für eine solche Form ist eine logarithmische Spirale, bei welcher alle durch den Pol gehenden Geraden die Spirale unter dem gleichen Tangentenwinkel schneiden.

Der Winkel α ist dabei bevorzugt in einem Bereich zwischen 20 und 50 Grad, bevorzugter 30 bis 40 Grad und ganz bevorzugt von 35 Grad gewählt. Der Winkel a, der über die gekrümmte Räumkante 14 hinweg im Wesentlichen konstant ist, ist dabei so gewählt, dass Sinkstoffe kontinuierlich in Richtung zu der Fangvorrichtung 6 hin transportiert werden, also vom Zentrum des Fermenterbehälters aus nach außen hin transportiert werden. Entsprechend muss der Winkel α so gewählt werden, dass zumindest die Anhaftungsreibung der Sinkstoffe an der Räumkante 14 bzw. an der Räumvorrichtung 10 bei einer Drehung der Räumvorrichtung 10 durch den durch den Winkel α bereit gestellten tangentialen Materialvorschub überwunden wird.

Andere Ausgestaltungen einer (gemäß den Ansprüchen zur Erfindung gehörigen) gekrümmten Räumkante 14 sind ebenfalls denkbar, solange der Effekt gegeben ist, dass die Sinkstoffe, die sich auf dem Boden 2 des Fermenterbehälters absetzen, durch die Rotation der Räumvorrichtung 10 nach außen transportiert werden, derart, dass sie an der Räumvorrichtung 10 nicht festhaften und andererseits aber eine erneute Aufwirbelung der sich bereits abgesetzten Sinkstoffe nicht auftritt.

Im Bereich der Fangvorrichtung 6 ist der sich in entgegengesetzter Richtung bzw. Steigung nach außen erstreckende Schenkel 62 zu erkennen, der quasi einen Potentialtopf für das Räumgut 5 derart ausbildet, dass das Räumgut 5 zuverlässig im Kreis herum gefördert wird, bis es über den Auswurfbereich 4 aus dem Fermentergehäuse abgeführt werden kann.

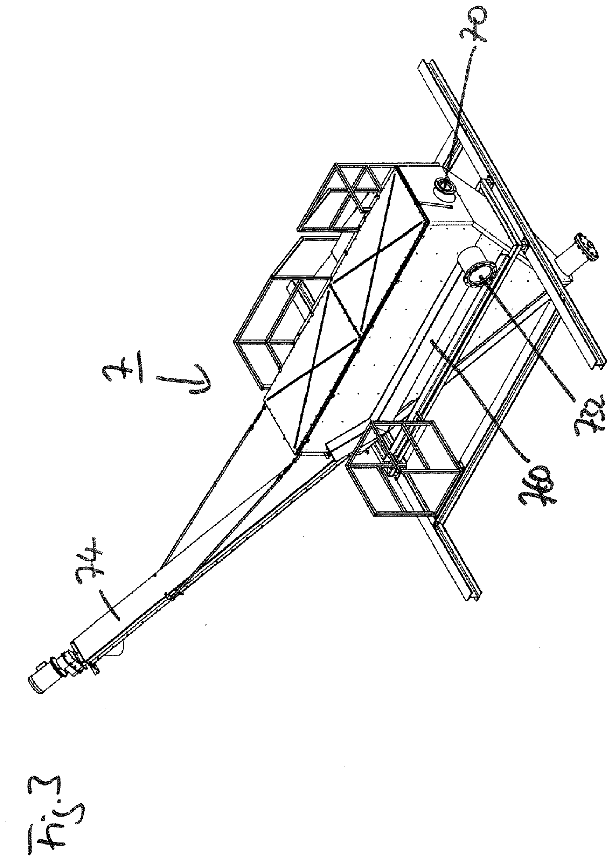

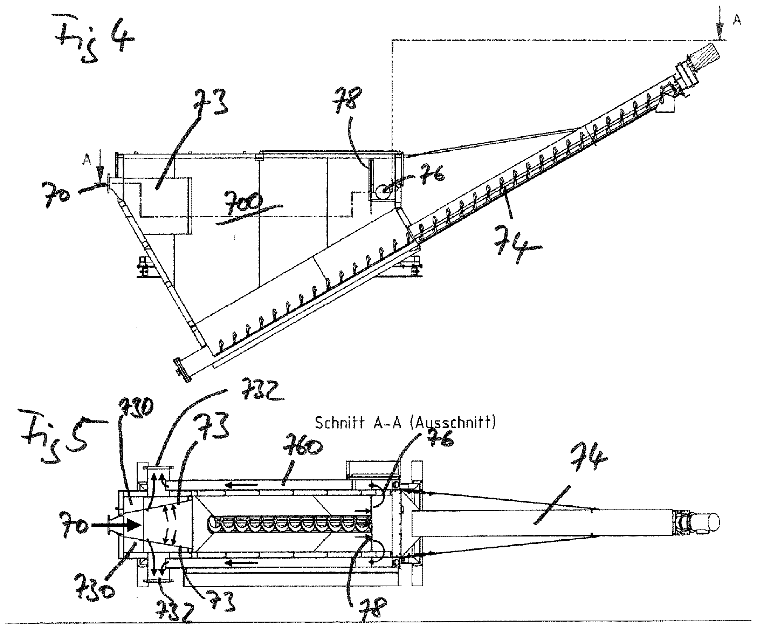

In den Figuren 3 bis 5 ist ein Schwerkraftabscheider 7 gezeigt, wobei dieser in Figur 3 in einer perspektivischen schematischen Zeichnung gezeigt ist, in Figur 4 in einer schematischen Schnittdarstellung und in Figur 5 in einer Draufsicht entlang der Linie A-A der Figur 4.

Der Schwerkraftabscheider 7 kommuniziert mit der Absaugvorrichtung derart, dass Räumgut, welches über den in den Figuren 1 und 2 gezeigten Auswurfbereich 4 abgesaugt wurde, über den Zulauf 70 in den Schwerkraftabscheider 7 eingebracht wird. Das Räumgut wird dabei typischerweise mit Wasser oder Gärschlamm gemischt eingebracht. Beim Pumpen des Räumguts in den Schwerkraftabscheider 7 staut sich das unter Druck über den Zulauf 70 in das Volumen 700 des Schwerkraftabscheiders 7 eintretende Räumgut und Flüssigkeitsgemisch am einstellbaren Wehr 78 auf. Zwei weitere Wehre 73 sind seitlich neben dem Zulauf 70 vorgesehen und bilden quasi zwei "Taschen" 730 aus, über die mittels eines ersten Auslaufes 732 ein erster Teilstrom des eintretenden Flüssigkeitsvolumens ablaufen kann, welcher bereits von schweren Sinkstoffen befreit ist, da diese unmittelbar nach dem Zulauf 70 absinken.

Ein zweiter Auslauf 76 ist vorhanden, über den ein zweiter Teilstrom, der über das Wehr 78 fließt, ablaufen kann, wobei dieser zweite Teilstrom bereits von feineren Sinkstoffen befreit ist, die durch die längere Verweildauer im Abscheidevolumen 700 abgesunken sind. Die Aufteilung der jeweiligen Teilströme erfolgt durch die Höheneinstellung des Wehrs 78.

Die Sinkstoffe setzen sich im Bereich der Räumschnecke 74 ab, welche das Räumgut aus dem Schwerkraftabscheider 7 austrägt.

Die feinen Schwebstoffe, welche beim Prozess des Abpumpens des Räumgutes durch die Öffnung 4 im Boden des Fermenterbehälters mit abgepumpt wurden, setzen sich nicht auf dem Boden ab und werden über die Ausgänge 732 und 76 aus dem Schwerkraftabscheider 7 wieder in den Fermenter zurückgeführt. Die Förderschnecke 74 fördert alle im Abscheidevolumen 700 abgesunkenen Sinkstoffe aus dem Schwerkraftabscheider 7 heraus.

Die beiden Teilströme, die über die Ausgänge 732 und 76 aus dem Schwerkraftabscheider ausgelaufen sind, werden mittels jeweils einer Leitung 760 auf jeder Seite des Schwerkraftabscheiders 7 wieder miteinander vereint und dann dem Fermenter wieder zugeführt.

### Bezugszeichenliste

- 1: Räumer
- 10: Räumvorrichtung
- 12: Anflanschbereich
- 14: Räumkante
- 100: Drehachse
- 2: Boden des Fermenterbehälters
- 20: Rand des Fermenterbehälters
- 3: Antrieb
- 4: Auswurfbereich
- 5: Räumgut
- 6: Fangvorrichtung
- 60: V-förmige Kante der Fangvorrichtung
- 62: Schenkel der Fangvorrichtung
- 64: Räumschild
- 7: Schwerkraftabscheider
- 70: Zulauf
- 73: festes Wehr
- 730: Tasche
- 732: erster Ablauf
- 74: Räumschnecke
- 76: zweiter Ablauf
- 760: Leitung
- 78: verstellbares Wehr
- 700: Abscheidevolumen

## Patentansprüche

1. Räumer (1) für einen Fermenter zum Fördern von Räumgut (5), insbesondere von Sinkstoffen, zu einem Auswurfbereich (4), wobei der Räumer eine Räumvorrichtung aufweist, welche sich von einer im Zentrum eines Fermenters anordenbaren Drehachse (100) aus erstreckt,
**dadurch gekennzeichnet, dass**
die Räumvorrichtung eine in einer senkrecht zu der Drehachse (100) liegenden Ebene gekrümmte Räumkante (14) aufweist, wobei die Räumkante (14) derart gekrümmt ist, dass der Winkel zwischen einem von der Drehachse ausgehenden Radiusstrahl und der an dem entsprechenden Schnittpunkt des Radiusstrahls mit der Räumkante (14) anliegenden Tangente über die Räumkante (14) hinweg im Wesentlichen konstant ist, wobei an dem der Drehachse (100) abgewendeten Ende der gekrümmten Räumkante (14) mindestens eine Fangvorrichtung (6) zum Fangen des entlang der Räumkante (14) geförderten Räumguts vorgesehen ist.

2. Räumer gemäß Anspruch 1, wobei die Räumvorrichtung in Form eines gekrümmten Räumarmes mit einer gekrümmten Räumkante (14) vorliegt.

3. Räumer gemäß Anspruch 1 oder Anspruch 2, wobei die Räumkante (14) die Form einer logarithmischen Spirale ausbildet.

4. Räumer gemäß einem der vorstehenden Ansprüche, wobei der im Wesentlichen konstante Winkel zwischen dem Radiusstrahl und der Tangente in einem Bereich zwischen 20 und 50 Grad, bevorzugter zwischen 30 und 40 Grad, besonders bevorzugt bei 35 Grad liegt.

5. Räumer gemäß einem der vorstehenden Ansprüche, wobei der im Wesentlichen konstante Winkel zwischen dem Radiusstrahl und der Tangente derart gewählt ist, dass Räumgut bei einer Rotation der Räumvorrichtung um die Drehachse (100) herum entlang der Räumkante (14) gefördert wird, und bevorzugt entlang der Räumkante (14) von der Drehachse (100) weg gefördert wird.

6. Räumer gemäß einem der vorstehenden Ansprüche, wobei sich die Räumkante (14) im Wesentlichen stetig entlang der Räumvorrichtung erstreckt.

7. Räumer gemäß einem der vorstehenden Ansprüche, wobei die Fangvorrichtung einen sich von dem der Drehachse (100) abgewendeten Ende der Räumvorrichtung ausgehenden Ende der Räumvorrichtung aus erstreckenden Schenkel (62) aufweist, welcher bevorzugt eine zur gekrümmten Räumkante (14) entgegengesetzte Steigung aufweist.

8. Räumer gemäß einem der vorstehenden Ansprüche, wobei die Fangvorrichtung (6) so an der Räumvorrichtung positioniert ist, dass sie mit mindestens einem Auswurfbereich (4) im Boden (2) eines Fermenterbehälters so korreliert, dass in der Fangvorrichtung (6) gefangenes Räumgut in den Auswurfbereich (4) eingebracht wird.

9. Räumer gemäß einem der vorstehenden Ansprüche, wobei der Räumer weiterhin eine Absaugvorrichtung umfasst, welche mit mindestens einem im Boden (2) eines Fermenterbehälters angeordneten Auswurfbereich (4) verbindbar ist.

10. Räumer gemäß Anspruch 9, wobei ein Schwerkraftabscheider (7) mit der Absaugvorrichtung kommuniziert, wobei der Schwerkraftabscheider ein Abscheidevolumen (700) und einen ersten Ablauf (732) zum Ablaufen eines Teilstromes, der von schweren Sinkstoffen befreit ist, umfasst, und wobei ein zweiter Ablauf (76) zum Ablaufen eines von leichteren Sinkstoffen befreiten Teilstroms vorhanden ist, wobei das Aufteilungsverhältnis der beiden Teilströme bevorzugt mittels eines Wehrs (78) eingestellt werden kann.

11. Räumer gemäß Anspruch 9 oder 10, wobei der erste Ablauf (732) unterhalb des zweiten Ablaufs (76) in dem Schwerkraftabscheider positioniert ist, wobei bevorzugt der erste Ablauf (732) mit dem zweiten Ablauf (76) über eine Leitung (760) kommuniziert.

12. Räumer gemäß einem der vorstehenden Ansprüche, wobei sich entlang der Drehachse (100) eine Antriebswelle zum Antrieb der Räumvorrichtung erstreckt und die Antriebswelle einen Umfang aufweist, der länger als die längste Faserlänge in dem Flüssigkeitsvolumen ist, wobei die Antriebswelle bevorzugt einen Radius zwischen 150 mm und 350 mm aufweist.

13. Fermenter mit einem Fermenterbehälter, wobei ein Räumer gemäß einem der vorstehenden Ansprüche am Boden (2) des Fermenterbehälters vorgesehen ist und die Drehachse (100) im Zentrum des Fermenterbehälters angeordnet ist, wobei sich die Räumvorrichtung von der Drehachse (100) aus in Richtung des Randes (20) des Fermenterbehälters erstreckt und die Räumvorrichtung um die Drehachse (100) herum rotierbar ist.

14. Verfahren zum Fördern von Räumgut in einem Fermenter zu einem Auswurfbereich (4) mit einem Räumer gemäß einem der vorstehenden Ansprüche, wobei das Räumgut durch Rotation der Räumvorrichtung entlang der Räumkante (14) gefördert wird.

15. Verfahren gemäß Anspruch 14, wobei eine Absaugvorrichtung intermittierend dann betrieben wird, wenn ein vorgegebener Bereich um mindestens einen Auswurfbereich (4) in einem Boden (2) eines Fermenterbehälters herum von der Räumvorrichtung überstrichen wird.

## Claims

1. Scraper (1) for a fermenter for conveying material (5) to be scraped, in particular settling sediments, to an ejection area (4), wherein the scraper has a broaching device which extends from a axis of rotation (100) which can be arranged in the centre of a fermenter,
**characterised in that**
the broaching device comprises a broaching edge (14) which is curved in relation to a plane being perpendicular to the axis of rotation (100), wherein the broaching edge (14) is curved in such a way that the angle between a radius beam emanating from the axis of rotation and the tangent lying at the corresponding point of intersection of the radius beam with the broaching edge (14) is substantially constant over the broaching edge (14), wherein at least one catching device (6) for catching the material conveyed along the broaching edge (14) is provided at the end of the curved broaching edge (14) being remote from the axis of rotation (100).

2. Scraper according to claim 1, wherein the broaching device is in the form of a curved broaching arm with a curved broaching edge (14).

3. Scraper according to claim 1 or claim 2, wherein the broaching edge (14) forms the shape of a logarithmic spiral.

4. Scraper according to one of the above claims, where the substantially constant angle between the radius beam and the tangent is in a range between 20 and 50 degrees, more preferably between 30 and 40 degrees, particularly preferably 35 degrees.

5. Scraper according to one of the above claims, wherein the substantially constant angle between the radius beam and the tangent is selected such that when the broaching device rotates about the axis of rotation (100), material to be cleared is conveyed along the broaching edge (14), and is preferably conveyed along the broaching edge (14) away from the axis of rotation (100).

6. Scraper according to one of the above claims, wherein the broaching edge (14) extends substantially continuously along the broaching device.

7. Scraper according to one of the above claims, wherein the catching device has a leg (62) extending from the outgoing end of the broaching device from the end remote from the axis of rotation of the broaching device which preferably has a slope opposite to the curved broaching edge (14).

8. Scraper according to one of the above claims, wherein the catching device (6) is positioned on the broaching device so as to correlate with at least one ejection area (4) in the bottom (2) of a fermenter container such that material caught in the catching device (6) is introduced into the ejection area (4).

9. Scraper according to one of the above claims, wherein the scraper further comprises a suction device which is connectable to at least one ejection area (4) arranged in the bottom (2) of a fermenter container.

10. Scraper according to claim 9, wherein a gravity separator (7) communicates with the suction device, wherein the gravity separator comprises a separation volume (700) and a first outlet (732) for discharging a partial flow freed from heavy settling sediments, and wherein a second outlet (76) is provided for discharging a partial flow freed from lighter settling sediments, wherein the division ratio of the two partial flows can preferably be adjusted by means of a weir (78).

11. Scraper according to claim 9 or 10, wherein the first outlet (732) is positioned below the second outlet (76) in the gravity separator, preferably the first outlet (732) communicating with the second outlet (76) via a conduit (760).

12. Scraper according to one of the above claims, wherein a drive shaft for driving the broaching device extends along the axis of rotation (100) and the drive shaft has a circumference which is longer than the longest fibre length in the liquid volume, the drive shaft preferably having a radius between 150 mm and 350 mm.

13. Fermenter comprising a fermenter container, wherein a scraper according to one of the above claims is provided on the bottom (2) of the fermenter container and the axis of rotation (100) is arranged in the centre of the fermenter container, wherein the broaching device extends from the axis of rotation (100) towards the edge (20) of the fermenter container and the broaching device is rotatable around the axis of rotation (100).

14. Method for conveying material to be scraped in a fermenter to an ejection area (4) with a scraper according to one of the above claims, wherein the material to be cleared is conveyed along the broaching edge (14) by rotation of the broaching device.

15. Method according to claim 14, wherein a suction device is operated intermittently when a predetermined area around at least one ejection area (4) in a bottom (2) of a fermenter container is swept by the broaching device.

## Revendications

1. Racleur (1) pour un fermenteur destiné à transporter des matières à racler (5), en particulier des sédiments de décantation, vers une zone d'éjection (4), le racleur comportant un dispositif de brochage qui s'étend à partir d'un axe de rotation (100) pouvant être disposé au centre d'un fermenteur,
**caractérisé en ce que**
le dispositif de brochage comprend une arête de brochage (14) qui est incurvée par rapport à un plan perpendiculaire à l'axe de rotation (100), dans lequel l'arête de brochage (14) est incurvée de telle manière que l'angle entre un faisceau de rayons émanant de l'axe de rotation et la tangente se trouvant au point d'intersection correspondant du faisceau de rayons avec l'arête de brochage (14) est sensiblement constant sur l'arête de brochage (14), dans lequel au moins un dispositif de capture (6) pour la capture de la matière transporté le long de l'arête de brochage (14) est prévu à l'extrémité de l'arête de brochage incurvé (14) qui est éloignée de l'axe de rotation (100).

2. Racleur selon la revendication 1, dans lequel le dispositif de brochage se présente sous la forme d'un bras de brochage avec une arête de brochage qui est incurvé (14).

3. Racleur selon la revendication 1 ou la revendication 2, dans lequel l' arête de brochage (14) forme la forme d'une spirale logarithmique.

4. Racleur selon l'une des revendications ci-dessus, où l'angle sensiblement constant entre le rayon du faisceau et la tangente se situe dans une plage comprise entre 20 et 50 degrés, de préférence entre 30 et 40 degrés, et plus particulièrement de préférence 35 degrés.

5. Racleur selon l'une des revendications ci-dessus, dans lequel l'angle sensiblement constant entre le rayon du faisceau et la tangente est choisi de telle sorte que lorsque le dispositif de brochage tourne autour de l'axe de rotation (100), la matière à déblayer est transportée le long de l'arête de brochage (14), et est de préférence transportée le long de l' arête de brochage (14) en s'éloignant de l'axe de rotation (100).

6. Racleur selon l'une des revendications ci-dessus, dans lequel l'arête de brochage (14) s'étend de manière sensiblement continue le long du dispositif de brochage.

7. Racleur selon l'une des revendications ci-dessus, dans lequel le dispositif de capture comporte une branche (62) s'étendant de l'extrémité sortante du dispositif de brochage à partir de l'extrémité éloignée de l'axe de rotation du dispositif de brochage qui présente de préférence une pente opposée à l'arête de brochage incurvée (14).

8. Racleur selon l'une des revendications ci-dessus, dans lequel le dispositif de capture (6) est positionné sur le dispositif de brochage de manière à être en corrélation avec au moins une zone d'éjection (4) dans le fond (2) d'un récipient de fermenteur de telle sorte que la matière capturée dans le dispositif de capture (6) soit introduite dans la zone d'éjection (4).

9. Racleur selon l'une des revendications ci-dessus, dans lequel le racleur comprend en outre un dispositif d'aspiration qui peut être relié à au moins une zone d'éjection (4) disposée dans le fond (2) d'un récipient de fermenteur.

10. Racleur selon la revendication 9, dans lequel un séparateur par gravité (7) est relié au dispositif d'aspiration, dans lequel le séparateur par gravité comprend un volume de séparation (700) et une première sortie (732) pour l'évacuation d'un flux partiel libéré de sédiments lourds de décantation, et dans lequel une deuxième sortie (76) est prévue pour l'évacuation d'un flux partiel libéré de sédiments plus légers de décantation, dans lequel le rapport de division des deux flux partiels peut être réglé de préférence au moyen d'un déversoir (78).

11. Racleur selon la revendication 9 ou 10, dans lequel la première sortie (732) est positionnée en dessous de la deuxième sortie (76) dans le séparateur par gravité, de préférence la première sortie (732) communiquant avec la deuxième sortie (76) par un conduit (760).

12. Racleur selon l'une des revendications ci-dessus, dans lequel un arbre d'entraînement pour l'entraînement du dispositif de brochage s'étend le long de l'axe de rotation (100) et l'arbre d'entraînement a une circonférence qui est plus longue que la plus grande longueur de fibre dans le volume de liquide, l'arbre d'entraînement ayant de préférence un rayon compris entre 150 mm et 350 mm.

13. Fermenteur comprenant un récipient de fermenteur, dans lequel un racleur selon l'une des revendications ci-dessus est prévu sur le fond (2) du récipient de fermenteur et l'axe de rotation (100) est disposé au centre du récipient de fermenteur, dans lequel le dispositif de brochage s'étend depuis l'axe de rotation (100) vers le bord (20) du récipient de fermenteur et le dispositif de brochage peut tourner autour de l'axe de rotation (100).

14. Procédé pour transporter des matières à racler dans un fermenteur vers une zone d'éjection (4) avec un racleur selon l'une des revendications ci-dessus, dans lequel les matières à racler sont transportées le long de l'arête de brochage (14) par rotation du dispositif de brochage.

15. Procédé selon la revendication 14, dans lequel un dispositif d'aspiration est actionné par intermittence lorsqu'une zone prédéterminée autour d'au moins une zone d'éjection (4) dans un fond (2) d'un récipient de fermenteur est balayée par le dispositif de brochage.
